# EUROPEAN PATENT APPLICATION

(11) **EP 4 033 247 A1**
(43) Date of publication of application: **27.07.2022**
(21) Application number: 21382052.5
(22) Date of filing: 22.01.2021
(51) Int. Cl.: G01N 33/569

(54) **MULTI-SPECIES IMMUNOASSAYS FOR DETECTING ANTIBODIES ANTI-SARS-COV-2 USING PROTEIN A FOR DETECTION OF CAPTURED ANTIBODIES**

(71) Applicant: Universidad De Zaragoza, 50009 Zaragoza (ES); Fundación Agencia Aragonesa Para La Investigación Y El Desarrollo (ARAID), 50018 Zaragoza (ES); Fundación Instituto de Investigación Sanitaria Aragón (IIS Aragón), 50009 Zaragoza (ES)
(72) Inventor: VILLANUEVA SAZ, Sergio, 50009 Zaragoza (ES); PÉREZ CABREJAS, María Dolores, 50009 Zaragoza (ES); FERNÁNDEZ CASASNOVAS, Antonio, 50009 Zaragoza (ES); TOBAJAS DE LA FUENTE, Ana Pilar, 50009 Zaragoza (ES); HURTADO GUERRERO, Ramón, 50018 Zaragoza (ES); PARDO JIMENO, Julian, 50018 Zaragoza (ES); SÁNCHEZ PANIAGUA, María Lourdes, 50009 Zaragoza (ES); VERDE ARRIBAS, María Teresa, 50009 Zaragoza (ES); PAÑO PARDO, José Ramón, 50009 Zaragoza (ES); SANTIAGO GARCÍA, Llipsy, 50009 Zaragoza (ES)
(74) Representative: Hoffmann Eitle

(57) **Abstract**

The present invention generally relates to a multi-specie *in vitro* method for detecting in a biological sample an antibody that binds to at least one epitope of the SARS-CoV-2 virus. The method comprises a step of contacting the sample with a SARS-CoV-2 protein or a fragment thereof comprising at least one epitope of the SARS-CoV-2 virus, and a step of detecting the formation of an antigen-antibody complex between the viral protein and the antibody present in the biological sample using protein A conjugated to a label.

## Description

### TECHNICAL FIELD OF THE INVENTION

The present invention relates to the medical field. In particular, the present invention relates to multi-species immunoassays using Protein A as conjugate to detect antibodies anti SARS-CoV-2 in a biological sample.

### BACKGROUND OF THE INVENTION

In late December 2019, several cases of pneumonia of unknown origin were reported from China, which in early January 2020 were announced to be caused by a novel coronavirus. The virus was denominated severe acute respiratory syndrome coronavirus 2 (SARS-CoV-2) and was identified as the causal agent of Coronavirus Disease 2019 (COVID-19). In a matter of a few months, the virus has spread around the world in record time, and COVID-19 was thus declared to be a pandemic by the World Health Organization (WHO) in March 2020.

Coronaviruses (CoV) are important in veterinary medicine such as transmissible gastroenteritis coronavirus (TGEV) or porcine epidemic diarrhea virus (PEDV) that can cause severe diarrhea in pigs. CoV are found in many different animal species, including humans. The first coronavirus described, named the Infectious Bronchitis Virus (IBV), was isolated from chicken embryos in 1937. Since then, numerous CoV have been detected in a wide variety of animals, including wild animals, farm animals, and pets. They are divided into the genera of the mammalian-associated α- and β-CoV and the bird-associated γ- and δ-CoV.

The spectrum of coronavirus diseases in animals ranges from mild to severe intestinal, respiratory, or systemic diseases. However, there are also many coronavirus infections in animals that do not appear to cause any symptoms. The presence of CoV in a wide variety of animal species strongly suggested that these pathogens are of zoonotic origin and are transmitted from wild animals to humans. In fact, as SARS-CoV-2 is highly similar to bat betacoronaviruses, or betacoronaviruses found in pangolins, it is suspected that one of those animal species may represent the original host of SARS-CoV-2 that is causing the 2020 pandemic.

During COVID-19 pandemic, some studies reported occasional infections of dogs and felines. Under experimental conditions, the susceptibility of additional species including ferrets, hamsters, and fruit bats has been demonstrated. Considering the presumed zoonotic origin of SARS-CoV-2, and the fact that this virus can also infect animals, including domestic animals, it is of pivotal importance to develop diagnostic tools that allow to identify in a fast and easy way the presence of antibodies against SARS-CoV-2 in humans but also in animals, which may be acting as natural reservoirs of this virus. Thus, it if of interest to identify susceptible animal species and to asses SARS-CoV-2 prevalence in this species, especially those in close contact with humans.

Currently, most of the animal infections are detected by real-time PCR, which is an assay that implies high cost of detection while it only provides information of infection during a very short time interval. Serological assays, which are cheaper, faster and easier to perform, represent a more feasible option to elucidate the host range of SARS-CoV-2 and the prevalence in susceptible species. However, these tests, such as ELISA tests, are based on the *in vitro* interaction between the virus and species-specific antibodies, not allowing high-throughput testing of different animal specimens.

In the present invention a multi species serological assay is provided.

### DESCRIPTION OF THE FIGURES

**Figure 1****:** ELISA using Protein A in 92 samples of seronegative pre-pandemic cats. Wells 1A and 1B correspond to human seropositive samples and well 1C to a human seronegative sample.
**Figure 2****:** ELISA using Protein A in 61 samples of seronegative ferrets. Wells 1A and 1B correspond to human seropositive samples and well 1C to a human seronegative sample.
**Figure 3****: (A)** ELISA using Protein A in seropositive and **(B)** in seronegative human samples. Negative control is a pre-pandemic sample.
**Figure 4****:** Regression plot for comparative ELISA tests: correlation between reference ELISA (using specific anti-IgG human antibody, X axis) and ELISA of the present invention (using Protein A, Y axis).
**Figure 5****:** Absorbance measurements of comparative ELISA tests: **(A)** ELISAs using Protein A versus ELISAS using Protein A/G in ferrets' samples infected with *Leishmania* and **(B)** ELISAs using Protein A versus ELISAS using Protein A/G in human, cats and ferrets' samples potentially infected and non-infected with SARS-CoV-2. H, human serum, C, cat serum; F, ferret serum. (-) seronegative, (+) seropositive.
**Figure 6****: (A)** Correlation between ELISAs using Protein A (X axis) and Protein A/G (Y axis) conjugates in 177 human samples and **(B)** ROD curve analysis.

### BRIEF DESCRIPTION OF THE INVENTION

In a **first** aspect, the invention relates to a multi-specie *in vitro* method for detecting in one biological sample an antibody that binds to at least one epitope of the SARS-CoV-2 virus, comprising:
a. contacting said at least one biological sample with at least one isolated SARS-CoV-2 protein, or at least one fragment of said isolated SARS-CoV-2 protein comprising at least one epitope of the SARS-CoV-2 virus, and
b. detecting the formation of an antigen-antibody complex between said virus protein or said fragment and an antibody present in said biological sample,
characterized in that the detection is performed by using a labelled protein, wherein the protein of the labelled protein consists of Protein A or a derivative thereof conjugated to a label.

The *in vitro* method according to the first aspect, wherein the at least one isolated SARS-CoV-2 protein is the SARS-CoV-2 Spike (S) protein of SEQ ID NO 1, or a variant or a fragment of SEQ ID NO 1 having at least 80% sequence identity to SEQ ID NO 1.

The *in vitro* method according to the first aspect, wherein the at least one fragment of said isolated SARS-CoV-2 S protein of SEQ ID NO 1 is the Receptor-binding domain (RBD) of SEQ ID NO 2, or a variant or a fragment of SEQ ID NO 2 having at least 80% sequence identity to SEQ ID NO 2.

The *in vitro* method according to the first aspect, wherein the RBD comprising at least one epitope of the SARS-CoV-2 virus, comprises at least 20 contiguous amino acid residues having at least 80% sequence identity with at least about 20 contiguous amino acid residues of SEQ ID NO.2.

The *in vitro* method according to the first aspect, wherein said at least one isolated SARS-CoV-2 RBD or fragment thereof is a recombinant expression product.

The *in vitro* method according to the first aspect, wherein said biological sample is a blood, plasma or serum sample.

The *in vitro* method according to the first aspect, wherein said biological sample is a blood, plasma or serum sample from a mammal, preferably a mammal selected from the group of human, mustelids or felines.

The *in vitro* method according to the first aspect, wherein said biological sample is a serum sample, and said SARS-CoV-2 RBD is as set forth in SEQ ID NO 2.

The *in vitro* method according to the first aspect, wherein said method is an *in vitro* diagnostic method for the detection of a subject having antibodies against the SARS-CoV-2 virus, wherein said subject is diagnosed as having antibodies against the SARS-CoV-2 virus if an antigen-antibody complex between said virus protein, or said fragment, and an antibody present in said biological sample is detected.

The *in vitro* method according to the first aspect, wherein said method is a method for screening a mammal having antibodies against the SARS-CoV-2 virus from those not having antibodies against the SARS-CoV-2 virus, wherein the mammal is preferably a mammal selected from the group of human, mustelids or felines.

In a second aspect, the present invention relates to a multi-specie *in vitro* kit suitable for detecting in a biological sample an antibody that binds to at least one epitope of the SARS-CoV-2 virus comprising:
a. at least one isolated SARS-CoV-2 protein, or at least one fragment of said isolated SARS-CoV-2 protein comprising at least one epitope of the SARS-CoV-2 virus, and
b. reagents for detecting the formation of antigen-antibody complex between said at least one isolated SARS-CoV-2 protein, or a fragment thereof, and at least one antibody present in a biological sample,
characterized in that the detection is performed by using a labelled protein, wherein the protein of the labelled protein consists of Protein A or a derivative thereof conjugated to a label, and wherein said at least one isolated protein or fragment thereof and said reagents are present in an amount sufficient to detect the formation of said antigen-antibody complex.

The kit according to the second aspect, wherein said at least one isolated SARS-CoV-2 protein is the Spike (S) protein of SEQ ID NO 1 or a variant or a fragment of SEQ ID NO 1 having at least 80% sequence identity to SEQ ID NO 1.

The kit according to the second aspect, wherein the at least one fragment of isolated SARS-CoV-2 S protein of SEQ ID NO 1 is the Receptor-binding domain (RBD) fragment of SEQ ID NO 2, or a variant or a fragment of SEQ ID NO 2 having at least 80% sequence identity to SEQ ID NO 2.

The kit according to the second aspect, wherein said at least one fragment of said isolated SARS-CoV-2 RBD comprising at least one epitope of the SARS-CoV-2 virus, is characterized by comprising at least 20 contiguous amino acid residues having at least 80% sequence identity with at least 20 contiguous amino acid residues of SEQ ID NO 2.

The kit according to the second aspect, wherein said at least one isolated SARS-CoV-2 RBD or derivative thereof is a recombinant expression product.

The kit according to the second aspect, wherein said at least one isolated SARS-CoV-2 RBD is the protein of SEQ ID NO 2.

The kit according to the second aspect, wherein said kit is an ELISA system comprising at least one isolated SARS-CoV-2 RBD of SEQ ID NO 2, to coat or coating a solid surface, preferably microtiter plate wells, and further consisting of Protein A conjugated to a label and one or more of the following reagents: blocking reagents for unbound sites to prevent false positive results; and substrates that react with the label, preferably the enzyme, to indicate a positive reaction.

The *in vitro* method according to the first aspect, wherein the formation of antigen-antibody complex is detected by radioimmunoassay (RIA), enzyme linked immunosorbent assay (ELISA), chemiluminescent or colorimetric enzyme linked immunosorbent assay (ELISA), immunofluorescence assay (IFA), dot blot or western blot.

The *in vitro* method according to the first aspect, wherein the formation of antigen-antibody complex is detected by enzyme linked immunosorbent assay (ELISA).

The *in vitro* method according to the first aspect, wherein said method is capable of detecting IgG, IgM and/or IgA, preferably IgG.

The *in vitro* method according to the first aspect, wherein the formation of antigen-antibody complex is detected by any of the methods identified above and the at least one virus protein or said fragment is adapted to detect IgG, IgM and/or IgA at a dilution of between 1:50 to 1:1000.

The *in vitro* method according to the first aspect, wherein said biological sample is contacted with at least one or more further SARS-CoV-2 immunogens or fragments thereof.

The *in vitro* method according to the first aspect, wherein said further immunogens are selected from the group consisting of nucleocapsid (N) proteins of SARS-CoV-2, and/or spike (S) domains including the S1 subunit.

The *in vitro* method according to the first aspect, wherein said biological sample is contacted with at least one or more further immunogens derived from at least one distinct isolated SARS protein.

In a **third** aspect, the present invention relates to a multi-specie ELISA **system** comprising at least one isolated SARS-CoV-2 protein or fragments thereof, as defined previously, to coat or coating a solid surface, preferably microtiter plate wells, further consisting of Protein A conjugated to a label, and optionally one or more of the following reagents: blocking reagents for unbound sites to prevent false positive results and substrates that react with the label to indicate a positive reaction.

The ELISA system according to the third aspect, wherein it further comprises additional reagents such as wash buffers, stop solutions and stabilizers.

In a **fourth** aspect, the present invention relates to the in *vitro* **use** of the kit or the ELISA system for use in the implementation of the method according to the first aspect.

### DESCRIPTION OF THE INVENTION

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

The term "about" when referred to a given amount or quantity is meant to include deviations of plus or minus five percent.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

A "fragment" of the SARS-CoV-2 protein according to the present invention is a partial amino acid sequence of the SARS-CoV-2 proteins or a functional equivalent of such a fragment. A fragment is shorter than the complete SARS-CoV-2 protein and is preferably between about 10, 50 or 100 and about 1000 amino acids long, more preferably between about 10, 50 or 100 and about 500 amino acids long, even more preferably between about 50 and about 250 amino acids long. A fragment of the SARS-CoV-2 protein also includes peptides having at least 15, 20 or 65 contiguous amino acid residues having at least about 70%, at least about 80%, at least about 90%, preferably at least about 95%, more preferably at least 98% sequence identity with at least about 15, 20 or 65 contiguous amino acid residues of SEQ ID NO. 1 or SEQ ID NO. 2 having about the same length as said peptides. Depending on the expression system chosen, the protein fragments may or may not be expressed in native glycosylated form.

A fragment that "corresponds substantially to" a fragment of the SARS-CoV-2 protein is a fragment that has substantially the same amino acid sequence and has substantially the same functionality as the specified fragment of the SARS-CoV-2 protein.

A fragment that has "substantially the same amino acid sequence" as a fragment of the SARS-CoV-2 protein typically has more than 90% amino acid identity with this fragment. Included in this definition are conservative amino acid substitutions.

By "Protein A or a derivative" is meant Staphylococcal protein A (SPA) or derivatives constructs that maintain its immunoglobulin binding properties. Protein A is a cell wall associated protein domain exposed on the surface of the Gram-positive bacterium, such as Staphylococcus aureus.

A "derivative" as used herein refers to a protein that, despite not having the very same amino acid sequence, has an equivalent function or antigenicity than the protein to which it is derivative. A "derivative of Protein A"as used herein is a peptide or a protein that comprises similar function as Protein A, that is, that binds to immunoglobulins.

"Antibodies" as used herein are polyclonal and/or monoclonal antibodies or fragments thereof, including recombinant antibody fragments, as well as immunologic binding equivalents thereof, which are capable of specifically binding to the SARS-CoV-2 S or RBD protein and/or to fragments thereof. The term "antibody" is used to refer to either a homogeneous molecular entity or a mixture such as a serum product made up of a plurality of different molecular entities. Recombinant antibody fragments may, e.g., be derived from a monoclonal antibody or may be isolated from libraries constructed from an immunized non-human animal.

By "antigen" or "immunogen" means a substance that induces a specific immune response in a host animal. The antigen may comprise a whole organism, killed, attenuated or live; a subunit or portion of an organism; a recombinant vector containing an insert with immunogenic properties; a piece or fragment of DNA capable of inducing an immune response upon presentation to a host animal; a polypeptide, a protein or a fragment thereof, an epitope, or any combination thereof.

"Epitope" as used herein refers to an antigenic determinant of a polypeptide. An epitope could comprise three amino acids in a spatial conformation which is unique to the epitope. Generally, an epitope consists of at least five such amino acids, and more usually consists of at least 8-10 such amino acids. Methods of determining the spatial conformation of such amino acids are known in the art.

The sensitivity of a test is the ability of a test to correctly identify those with the disease (true positive rate), whereas the specificity of a test is the ability of the test to correctly identify those without the disease (true negative rate). "Sensitivity" as used herein in the context of testing a biological sample is the percentile of the number of true positive anti-SARS-CoV-2 samples divided by the total of the number of true positive anti-SARS-CoV-2 samples plus the number of false negative anti-SARS-CoV-2 samples. "Specificity" as used herein in the context of testing a biological sample is the percentile of the number of true negative anti-SARS-CoV-2 samples divided by the total of the number of true negative anti-SARS-CoV-2 samples plus the number of false positive samples.

"Detection rate" as used herein in the context of antibodies specific for the SARS-CoV-2 virus is the percentile of the number of SARS-CoV-2 positive samples in which the antibody was detected divided by the total number of SARS-CoV-2 positive samples tested. "Overall detection rate" as used herein refers to the virus detection obtained by detecting IgM and/or IgG.

The terms "sequence identity" or "percent identity" in the context of two or more polypeptides or proteins refers to two or more sequences or subsequences that are the same ("identical") or have a specified percentage of amino acid residues that are identical ("percent identity") when compared and aligned for maximum correspondence with a second molecule, as measured using a sequence comparison algorithm (e.g., by a BLAST alignment, or any other algorithm known to persons of skill), or alternatively, by visual inspection.

A protein or peptide of the present invention has substantial identity with another if, optimally aligned, there is an amino acid sequence identity of at least about 60% identity with a naturally-occurring protein or with a peptide derived therefrom, usually at least about 70% identity, more usually at least about 80% identity, preferably at least about 90% identity, and more preferably at least about 95% identity, and most preferably at least about 98% identity. Identity means the degree of sequence relatedness between two polypeptide or two polynucleotides sequences as determined by the identity of the match between two strings of such sequences, such as the full and complete sequence. Identity can be readily calculated. While there exist a number of methods to measure identity between polypeptide sequences, the term "identity" is well known to skilled artisans.

### DESCRIPTION

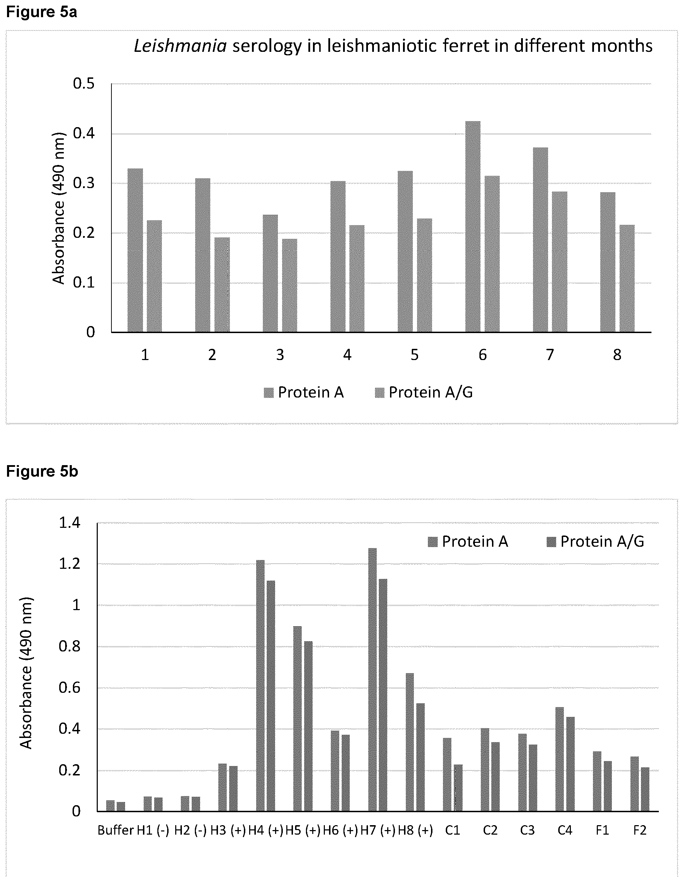

Multiple tests to detect antibodies against SARS-CoV-2 are becoming available. However, these assays are usually designed to detect species-specific antibodies, which means that their use is limited to the animal species they are designed to react with, usually humans. Thus, when it comes to the rapid and effective study of the seroprevalence of a pathogen in animals in general (not only humans), these antibody tests are not useful since they do not work with biological samples from different animal species and, in order to achieve testing in different animals, a specific test designed for the specific animal species needs to be used. Alternatively, a species-specific immunoassay can be adapted to react to a different animal sample by changing the secondary antibody conjugate to one that is specific of the other species, but this comes with the risk that the sensitivity and specificity of the test may be compromised and in this case the assay would still not work for different animals samples at the same time (the secondary antibody would need to be changed every time a sample from a different origin is tested). In order to solve this problem, the authors of the present invention have developed an immunoassay that can detect antibodies anti-SARS-CoV-2 virus present in different biological samples from different animal origin without changing any reagent, making this immunoassay universal (multi-species). As shown in Figure 5b, ELISAs were performed according to the present invention and antibodies against SARS-CoV-2 virus in human, cats and ferret's samples were detected. Therefore, the first advantage of the present invention is that it can be used with biological samples from different animal species, making it a high-throughput assay that solves the problem of having to use different (specific) immunoassays for each species that wants to be tested.

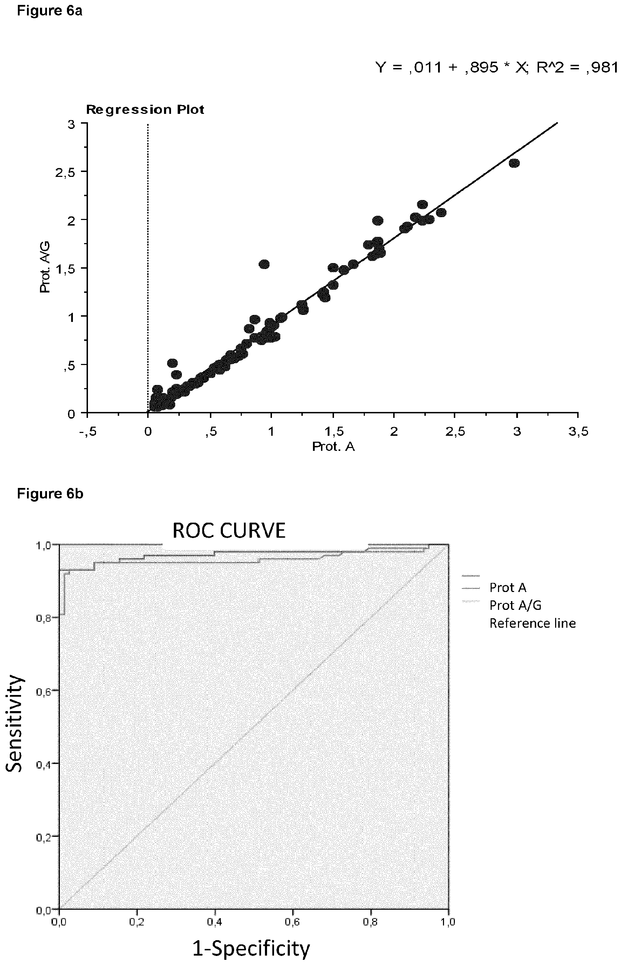

Further, the immunoassay of the present invention not only works with biological samples from different origins (different animals) but it also presents increased sensitivity and specificity in comparison with other routine immunoassays. This is shown in Figure 6 where the assay of the present invention which uses Protein A was compared to state-of-the art assays that use Protein A/G. It is noted that, in assays that imply antibody binding, the combined use of Protein A/G is expected to improve and broaden the affinity to immunoglobulins since Protein A/G contains four Protein A binding domains plus the two Protein G binding domains. However, a second aspect that was surprisingly found in the present invention is that the immunoassays disclosed herein using Protein A alone showed an increased sensitivity and specificity over assays using Protein A/G, presenting lower background signal, especially when seronegative samples are used (as shown in Table 3, p=0.0007). Thus, the immunoassay of the present invention represents an improvement (in sensitivity and specificity) over state of the art techniques, allowing a better discrimination of positive samples that have low absorbance signal and therefore reducing the rate of false negatives.

Thus, generally, the present invention provides a multi-species assay to detect and study the seroprevalence of SARS-CoV-2 in different animals with high sensitivity and specificity.

In a **first aspect** of the present invention, it is provided a multi-specie *in vitro* method for detecting in one biological sample an antibody that binds to at least one epitope of the SARS-CoV-2 virus, comprising:
a. contacting said at least one biological sample with at least one isolated SARS-CoV-2 protein, or at least one fragment of said isolated SARS-CoV-2 protein comprising at least one epitope of the SARS-CoV-2 virus, and
b. detecting the formation of an antigen-antibody complex between said virus protein or said fragment and an antibody present in said biological sample,
characterized in that the detection is performed by using a labelled protein, wherein the protein of the labelled protein consists of or comprises of Protein A or a derivative thereof conjugated to a label.

By "a labelled protein" is meant a protein to which a reporter group (label) is attached to so that the detection of the protein can be carried out, for instance by chemical or fluorescence means.

By "Protein A or a derivative" is meant Staphylococcal protein A (SPA) or derivatives constructs that maintain its immunoglobulin binding properties. Protein A is a cell wall associated protein domain exposed on the surface of the Gram-positive bacterium, such as *Staphylococcus aureus.* Protein A consists of three different regions: region S, which is the signal sequence that is processed during secretion, five homologous IgG binding domains E, D, A, B and C and a cell-wall anchoring region. Since the 11 residues of Protein A involved in the Fab interaction are located on the second and third helices, a derivative of Protein A that may be used in the present invention is a Protein A fragment comprising said residues. Further, peptides that mimic the interaction between Protein A and the Fc part of the immunoglobulins where Protein A binds are also included herein. Also included are the improvements of one or more of the immunoglobulin-binding domains present in Protein A, for instance by introducing site specific mutations to increase its stability or its binding affinity, or by introducing repetitive binding domains to increase the affinity of Protein A to the immunoglobulin.

In an embodiment, the amount of Protein A to be used ranges from 10-1 mg/ml, preferably in a concentration of 5 mg/ml, 4 mg/ml, 3 mg/ml, 2 mg/ml or 1 mg/ml. In a further preferred embodiment, the Protein A is used in a concentration of 100-10 ng/ml, preferably 50ng/ml.

The "Protein A or a derivative thereof" as defined herein may be a commercially available Protein A, or an *in-house* produced Protein A. Methods for production and purification of Protein A are well known to the skilled artisan. The Protein A or a derivative thereof may be a recombinant expression product.

As stated above, Protein A or a derivative thereof is conjugated to a label to allow the detection of the complex formed by the Protein A or the derivative thereof with the antibody that binds to at least one epitope of the SARS-CoV-2 virus. In an embodiment, the reporter or label includes radioisotopes, fluorophores and enzymes. In a preferred embodiment, the Protein A or derivative thereof is conjugated to an enzyme, for instance horseradish peroxidase.

In an embodiment, the method of the first aspect is characterized in that the detection is performed by using a labelled protein, wherein the protein of the labelled protein comprises Protein A. In an embodiment, the protein of the labelled protein is Protein A and, optionally, another protein with immunoglobulin-binding properties, such as Protein G or Protein L, either as a recombinant fusion protein or as a combination of both proteins individually. In a preferred embodiment, the method of the first aspect is characterized in that the detection is performed by using a labelled protein, wherein the protein of the labelled protein consists of Protein A.

In an embodiment, the "at least one isolated SARS-CoV-2 protein" may be any protein present in SARS-CoV-2 virus. SARS-CoV-2 virus has a total of 11 genes with 11 open reading frames (ORFs): ORF1ab, ORF2 (Spike protein), ORF3a, ORF4 (Envelope protein), ORF5 (Membrane protein), ORF6, ORF7a, ORF7b, ORF8, ORF9 (Nucleocapsid protein), and ORF10. The four structural proteins of coronaviruses are: S protein, M protein, E protein, and N protein. The S protein is a glycoprotein that mediates attachment of the virus to the host cell. The SARS coronavirus membrane (M) protein is an integral membrane protein that plays an important role in viral assembly. The E protein plays multiple roles in the viral replication cycle: viral assembly, virion release, and viral pathogenesis. The nucleocapsid (N) protein of coronaviruses is a structural protein that binds directly to viral RNA and providing stability.

In a preferred embodiment, the at least one isolated SARS-CoV-2 protein is a structural protein, preferably located on the surface of the virus, such as nucleocapsid protein (N), envelop protein (E), membrane protein (M), Spike protein (S), including the S1 subunit and/or derivatives and/or fragments thereof. The receptor-binding domain (RBD) is comprised within the S protein, particularly within the S1 subunit, and binds to angiotensin-converting enzyme 2 (ACE2), which serves as an entry receptor. RBD is an immunodominant epitope and the target of 90% of the neutralizing activity present in SARS-CoV-2 immune sera.

In a still preferred embodiment of the first aspect, the at least one isolated SARS-CoV-2 protein is the Spike (S) protein of Sars-CoV-2 virus, or a variant or a fragment thereof. More preferably, the at least one isolated SARS-CoV-2 S protein is the S protein of SEQ ID NO 1 or a variant of SEQ ID NO 1 having at least 80%, 85%, 90% or 95% sequence identity to SEQ ID NO 1, e.g., 95%, 96%, 97%, 98%, or 99%, sequence identity to SEQ ID NO 1.

In a further preferred embodiment of the first aspect, the at least one fragment of said isolated SARS-CoV-2 S protein of SEQ ID NO 1 is the Receptor-binding domain (RBD) or a variant or a fragment thereof. More preferably, the at least one fragment of said isolated SARS-CoV-2 S protein of SEQ ID NO 1 is the RBD of SEQ ID 2 or a variant of SEQ ID NO 2 having at least 80%, 85%, 90% or 95% sequence identity to SEQ ID NO 2, e.g., 95%, 96%, 97%, 98%, or 99%, sequence identity to SEQ ID NO 2.

In another preferred embodiment of the first aspect, the at least one isolated SARS-CoV-2 S or RBD proteins comprising at least one epitope of the SARS-CoV-2 virus, comprise at least 10, 15, 20 or 65 contiguous amino acid residues having at least about 70%, at least about 80%, at least about 90%, preferably at least about 95%, more preferably at least 98% sequence identity with at least about 10, 15, 20 or 65 contiguous amino acid residues of SEQ ID NO 1 or SEQ ID NO 2, respectively. Preferably, said at least one fragment of said isolated SARS-CoV-2 S or RBD proteins have a sensitivity of more than about 70%, at least about 80%, at least about 90%, preferably at least about 95%, more preferably at least 98% or 99%. Preferably, said at least one fragment of said isolated SARS-CoV-2 S or RBD proteins have a specificity of more than about 70%, at least about 80%, at least about 90%, preferably at least about 95%, more preferably at least 98% or 99%. More preferably, said at least one fragment of said isolated SARS-CoV-2 S or RBD protein has a detection rate or an overall detection rate of more than about 65%, at least about 70%, at least about 80%, at least about 90%, preferably at least about 95%, more preferably at least 98% or 99%.

In another preferred embodiment of the first aspect or of any of its preferred embodiments, said at least one isolated SARS-CoV-2 S or RBD proteins or fragment thereof are recombinant expression products.

In still another preferred embodiment of the first aspect or of any of its preferred embodiments, said method is adapted to detect IgG, IgM and/or IgA. Preferably, said method is adapted to detect IgG. In another preferred embodiment said method is adapted to detect IgG, IgM and/or IgA at a dilution of a fluid biological sample, preferably blood, more preferably serum or plasma, of about 1:100, about 1:800, about 1:900, about 1:1000, about 1:1100 up to about 1:1200, 1:800 or 1:3200. In another preferred embodiment, the method according to the present invention is able to detect IgG, IgM and/or IgA at a dilution of a fluid biological sample, preferably serum, of about 1:50, about 1:100, about 1:200 up to about 1:400, or about 1:1000.

In still another preferred embodiment of the first aspect or of any of its preferred embodiments, appropriate biological samples to practice the method include, but are not limited to, mouth gargles, any biological fluids, virus isolates, tissue sections, wild and laboratory animal samples. Preferably, said biological sample is a blood, plasma or serum sample isolated from an animal.

In an embodiment, the animal is preferably a mammal. In a preferred embodiment, the mammal is selected from the group of human, mustelids or felines. In a further preferred embodiment, the mammal is selected from the group of cats, dogs, rats, cows, goats, sheeps, horses, pigs, ferrets, human, rabbit, guinea pig, bovine and/or mouse. More preferably, the mammals are cats, human and/or ferrets. Preferably, said biological sample is a serum or sera sample and said SARS-CoV-2 RBD is as set forth in SEQ ID NO 2.

In still another preferred embodiment of the first aspect or of any of its preferred embodiments, said method is an *in vitro* diagnostic method for the detection of a subject having antibodies against the SARS-CoV-2 virus, wherein said subject is preferably a mammal, more preferably a human being, a mustelid, preferably a ferret, or a feline, preferably a cat, and wherein said subject is diagnosed as having antibodies against the SARS-CoV-2 virus if an antigen-antibody complex between said virus protein, or said fragment, and an antibody present in said biological sample is detected.

In a preferred embodiment, said *in vitro* diagnostic method according to the present invention, will be able to additionally detect a wide array of stages of a SARS-CoV-2 infection. In still another preferred embodiment, said diagnostic method will be able to detect early stages of a SARS-CoV-2 infection. In another preferred embodiment, said diagnostic method will be able to detect early stages of infection by being able to detect IgM. In another preferred embodiment, said diagnostic method will be able to detect later stages of infection or a past infection by being able to detect IgG. In another preferred embodiment, said diagnostic method will be able to detect early stages of infection by being able to detect very low concentrations of antibodies. Accordingly, in a preferred embodiment the diagnostic method is adapted to detect antibodies against a SARS-CoV-2 virus less than about 50 days after the onset of symptoms, preferably less than about 40, less than about 30, less than about 25, less than about 20, less than about 15, less than about 12, less than about 10, less than about 9, less than about 8, less than about 7, less than about 6, less than about 5 less, or than about 4 days after the onset of symptoms.

In still another preferred embodiment of the first aspect or of any of its preferred embodiments, said method is an *in vitro* method for screening a mammal having antibodies against the SARS-CoV-2 virus from those not having antibodies against the SARS-CoV-2 virus, wherein the mammal is preferably a mammal selected from the group of human, mustelids or felines. Preferably, the mammal is selected from the group of cats, dogs, rats, cows, goats, sheeps, horses, pigs, ferrets, human, rabbit, guinea pig, bovine and/or mouse. More preferably, the mammals are cats, human and/or ferrets.

In another embodiment of the first aspect or its preferred embodiments, the existence of antigen-antibody binding can be detected via methods well known in the art. Preferably, these methods include enzyme-linked immunosorbent assays (ELISA) and dot blotting. Both of these methods are relatively easy to use and are high throughput methods. ELISA, in particular, has achieved high acceptability with clinical personnel. ELISA, preferably based in chemiluminescent or colorimetric methods, is also highly sensitive. However, any other suitable method to detect antigen-antibody complexes such as, but not limited to, standardized radio immunoassays (RIA), or immunofluorescence assays (IFA), dot blot, or western blot also can be used. A specially preferred method is the ELISA assay, more preferably a colorimetric or chemiluminescent enzyme linked immunosorbent assay (ELISA).

In still another preferred embodiment of the first aspect or of any of its preferred embodiments, the method of detection is an ELISA assay by using the ELISA system as described later in the present specification.

In another embodiment of the first aspect or of any of its preferred embodiments, the biological sample is contacted with at least one or more further SARS-CoV-2 immunogens or fragments thereof, wherein preferably said immunogens are selected from the group consisting of nucleocapsid (N) proteins of SARS-CoV-2 and/or spike (S) domains including the S1 subunit and RBD of SARS-CoV-2. In still another preferred embodiment of the first aspect or of any of its preferred embodiments, said biological sample is contacted with at least one or more further immunogens derived from at least one distinct isolated SARS protein.

A **second aspect** of the invention refers to a multi-specie *in vitro* kit suitable for detecting in a biological sample an antibody that binds to at least one epitope of the SARS-CoV-2 virus comprising:
a. at least one isolated SARS-CoV-2 protein, or at least one fragment of said isolated SARS-CoV-2 protein comprising at least one epitope of the SARS-CoV-2 virus, and
b. reagents for detecting the formation of antigen-antibody complex between said at least one isolated SARS-CoV-2 protein, or a fragment thereof, and at least one antibody present in a biological sample,
characterized in that the detection is performed by using a labelled protein, wherein the protein of the labelled protein consists of or comprises of Protein A or a derivative thereof conjugated to a label, and
wherein said at least one isolated protein or fragment thereof and said reagents are present in an amount sufficient to detect the formation of said antigen-antibody complex.

The Protein A or a derivative thereof of the second aspect is conjugated to a label to allow the detection of the complex formed by the Protein A or the derivative thereof with the antibody that binds to at least one epitope of the SARS-CoV-2 virus. In an embodiment, the label includes radioisotopes, fluorophores and enzymes. In a preferred embodiment, the Protein A or derivative thereof is conjugated to an enzyme, such as horseradish peroxidase.

In a preferred embodiment of the second aspect of the invention, said at least one isolated SARS-CoV-2 protein is the S protein, preferably the S protein of SEQ ID NO 1 or a variant of SEQ ID NO 1 having at least 80%, 85%, 90% or 95% sequence identity to SEQ ID NO 1, e.g., 95%, 96%, 97%, 98%, or 99%, sequence identity to SEQ ID NO 1.

In a further preferred embodiment of the second aspect of the invention, said at least one fragment of isolated SARS-CoV-2 S protein of SEQ ID NO 1 is the RBD of SEQ ID NO 2 or a variant of SEQ ID NO 2 having at least 80%, 85%, 90% or 95% sequence identity to SEQ ID NO 2, e.g., 95%, 96%, 97%, 98%, or 99%, sequence identity to SEQ ID NO 2.

In another preferred embodiment of the second aspect of the invention, said at least one fragment of said isolated SARS-CoV-2 S or RBD proteins comprising at least one epitope of the SARS-CoV-2 virus, comprises at least 10, 15, 20 or 65 contiguous amino acid residues having at least about 70%, at least about 80%, at least about 90%, preferably at least about 95%, more preferably at least 98% sequence identity with at least about 10, 15, 20 or 65 contiguous amino acid residues of SEQ ID No. 1 or SEQ ID NO 2, respectively.

In another preferred embodiment of the second aspect of the invention, said reagents are capable of detecting IgG, IgM and/or IgA. Preferably, said reagents are capable of detecting IgG. In another preferred embodiment said reagents are capable of detecting IgG at a dilution of about 1:100, about 1:800, about 1:900, about 1:1000, about 1:1100 up to about 1:1200. In another preferred embodiment, said reagents are capable of detecting IgM at a dilution of about 1:50, about 1:100, about 1:500, or up to about 1:1000.

In a particularly preferred embodiment, the kit is suitable for performing a radioimmunoassay (RIA), enzyme linked immunosorbent assay (ELISA) preferably a chemiluminescent or colorimetric enzyme linked immunosorbent assay (ELISA), immunofluorescence assay (IFA), dot blot, or western blot. Preferably, the kit is an ELISA system.

In the context of the present invention, the ELISA (enzyme-linked immunosorbent assay) system is preferably understood as a plate-based assay technique designed for detecting and quantifying antibodies against at least one isolated SARS-CoV-2 protein, preferably S or RBD proteins, or fragment thereof. In this ELISA, the at least one isolated SARS-CoV-2 S or RBD proteins or fragment thereof must be immobilized to a solid surface and then exposed to the biological sample to form a complex. Detection is accomplished by any techniques well known in the art.

ELISAs, according to the present invention, are typically performed in 96-well (or 384-well) polystyrene plates, which will passively bind the at least one isolated SARS-CoV-2 protein or fragments thereof, preferably the S or RBD proteins. The binding and immobilization of reagents makes ELISAs simple to design and perform. Having the reactants of the ELISA immobilized to the microplate surface enables easy separation of bound from non-bound material during the assay. This ability to wash away non-specifically bound materials makes the ELISA a powerful tool for measuring specific analytes within a crude preparation.

In a preferred embodiment of the second aspect of the invention, the ELISA system comprises at least one isolated SARS-CoV-2 protein or fragments thereof, preferably the S of SEQ ID 1 or RBD of SEQ ID 2, to coat or coating a solid surface, preferably microtiter plate wells, the ELISA system further consisting of Protein A conjugated to a label and used to detect the bound complex. Optionally one or more of the following reagents may be included: blocking reagents for unbound sites to prevent false positive results and substrates that react with the label, preferably the enzyme, to indicate a positive reaction. In addition to the procedure reagents, additional reagents such as wash buffers, stop solutions and stabilizers can enhance the quality of the ELISA assay. When choosing individual reagents, or complete kits, it is helpful to know the sensitivity required and whether one is trying to detect an analyte or the antibody response to it.

In still another preferred embodiment of the second aspect of the invention, said at least one isolated SARS-CoV-2 S or RBD protein or fragment or derivative thereof is a recombinant expression product.

In still another preferred embodiment of the second aspect of the invention, said at least one isolated SARS-CoV-2 S protein is the protein of SEQ ID NO 1. In still another preferred embodiment of the second aspect of the invention, said at least one isolated SARS-CoV-2 RBD protein is the protein of SEQ ID NO 2.

A **third aspect** of the present invention relates to a multi-specie ELISA system comprising at least one isolated SARS-CoV-2 protein or fragments thereof, as defined previously in the first aspect, to coat or coating a solid surface, preferably microtiter plate wells, further consisting of or comprising of Protein A conjugated to a label, and optionally one or more of the following reagents: blocking reagents for unbound sites to prevent false positive results and substrates that react with the label to indicate a positive reaction.

In a preferred embodiment, the ELISA system further comprises additional reagents such as wash buffers, stop solutions and stabilizers.

A **fourth aspect** of the invention refers to the *in vitro* use of kit of the second aspect of the invention or of any of its preferred embodiments, for implementing any of the methods disclosed in the first aspect of the invention.

The methods, systems and the uses of the present invention that are described above are those which presently appear most attractive. However, the foregoing disclosures of embodiments of the invention and uses therefor have been given merely for purposes of illustration and not to limit the invention. Thus, the invention should be considered to include all embodiments falling within the scope of the claims following the Example section and any equivalents thereof.

The following examples are merely illustrative and should not be construed to limit in any way the invention as set forth in the claims which follow.

### SEQUENCE LISTING

Amino acid sequence SARS-CoV-2 Spike protein (SEQ ID NO 1):
Amino acid sequence SARS-CoV-2 RBD protein (SEQ ID NO 2):

RVQPTESIVRFPNITNLCPFGEVFNATRFASVYAWNRKRISNCVADYSVLYNSASFSTFKCY
GVSPTKLNDLCFTNVYADSFVIRGDEVRQIAPGQTGKIADYNYKLPDDFTGCVIAWNSNNLD
SKVGGNYNYLYRLFRKSNLKPFERDISTEIYQAGSTPCNGVEGFNCYFPLQSYGFQPTNGV
GYQPYRVVVLSFELLHAPATVCGPKKSTNLVKNKCVNF

### EXAMPLES

### EXAMPLE 1

### Materials and Methods

Antibodies to SARS-CoV-2 were determined by an indirect ELISA for the detection of IgG specific for Receptor Binding Domain (RBD) of Spike. Ninety-six-well plates were coated overnight, at 4 °C with 50 µl/well of RBD protein at 1µg/ml in phosphate buffered saline (PBS). Subsequently, the coating solution was removed and the plate was washed three times with 200 µL per well of PBS containing 0.05% Tween 20 (PBST). After, 300 µl of dry skimmed milk at 3% in PBST were added to each well as blocking solution. Plate was incubated with blocking solution for 1 h at 37 °C in a moist chamber. A volume of 100 µl of cat sera, diluted 1:100 in 1% dry skimmed milk in PBST (PBST-M), was added to each well. The plates were incubated for 1 hour at 37 °C in a moist chamber. After washing the plates for 30 seconds 6 times with PBST followed by 1 wash with PBS for 1 min, 100 µl/well of Protein A conjugated to horseradish peroxidase (Thermo Fisher Scientific, Waltham, Massachusetts, USA) (5 mg/ml diluted 1:100000 in PBST-M) was added per well. The plates were incubated for 1 hour at 37 °C in the moist chamber and were washed again with PBST and PBS as described above. The substrate solution (ortho-phenylene-diamine) dissolved in stable peroxide substrate buffer (Thermo Fisher Scientific, Waltham, Massachusetts, USA) was added at 100 µl per well and developed for 20 ±5 min at room temperature in the dark. The enzymatic reaction was stopped by adding 100 µl of 2.5 M H2SO4 to each well. Absorbance values were read at 492 nm in an automatic microELISA reader (Microplate Photometer Biosan Hipo MPP-96, Riga, Latvia). As a positive control, each plate included serum from a human patient diagnosed with COVID, confirmed by a molecular test and a commercial quantitative ELISA, and serum from a healthy, non-infected cat obtained prior to pandemic COVID-19 situation as negative control. The same positive and negative sera were used for all assays and plates, with a constant inter-assay variation of < 10%. Plates with an inter-assay variation of >10% were discarded.

### EXAMPLE 2

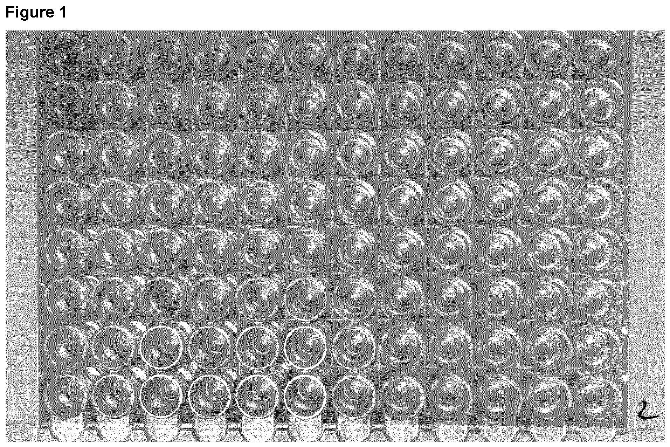

To evaluate the background signal that the ELISA assay of the present invention develops when animal sera is assayed, ELISA assays were performed as described in Example 1 above. As samples, 92 cat and 61 ferret sera obtained before 2019 COVID pandemic were used, and thus they were not infected with SARS-CoV-2. The results are shown in Figures 1 (cats) and 2 (ferrets). The cut-off absorbance point for negative sera is 0.47 in cat's samples and 0.30 in ferret's samples, showing that the ELISA of the present invention develops weak signal in the presence of sera from animals that are negative for antibodies against SARS-CoV-2.

The absorbance values obtained are represented below:

| **490 nm** | **1** | **2** | **3** | **4** | **5** | **6** | **7** | **8** | **9** | **10** | **11** | **12** |
|---|---|---|---|---|---|---|---|---|---|---|---|---|
| **A** | 0.7063 | 0.1564 | 0.2819 | 0.1006 | 0.0762 | 0.1139 | 0.1564 | 0.1186 | 0.1093 | 0.0587 | 0.0977 | 0.1324 |
| **B** | 1.1662 | 0.2046 | 0.4244 | 0.1466 | 0.1802 | 0.0994 | 0.0924 | 0.1201 | 0.0658 | 0.1066 | 0.0860 | 0.1438 |
| **C** | 0.1685 | 0.1328 | 0.4344 | 0.1044 | 0.1362 | 0.0821 | 0.0838 | 0.144 | 0.1428 | 0.1011 | 0.0581 | 0.0593 |
| **D** | 0.5247 | 0.1638 | 0.1776 | 0.1019 | 0.1116 | 0.1131 | 0.1498 | 0.1649 | 0.0817 | 0.1810 | 0.1101 | 0.0804 |
| **E** | 0.142 | 0.1130 | 0.2211 | 0.0608 | 0.1066 | 0.0931 | 0.0952 | 0.1000 | 0.1461 | 0.1156 | 0.0865 | 0.0982 |
| **F** | 0.2128 | 0.1405 | 0.4915 | 0.0658 | 0.0605 | 0.098 | 0.1006 | 0.0649 | 0.1419 | 0.0702 | 0.1264 | 0.0595 |
| **G** | 0.1647 | 0.1042 | 0.1424 | 0.1204 | 0.081 | 0.1223 | 0.0953 | 0.1749 | 0.0978 | 0.1375 | 0.2070 | 0.0582 |
| **H** | 0.087 | 0.0854 | 0.116 | 0.0911 | 0.0778 | 0.1366 | 0.1604 | 0.0853 | 0.0719 | 0.1031 | 0.0718 | 0.0498 |

Corresponding to Figure 1 (cats)

| **490 nm** | **1** | **2** | **3** | **4** | **5** | **6** | **7** | **8** |
|---|---|---|---|---|---|---|---|---|
| **A** | 0.5646 | 0.0986 | 0.1759 | 0.1218 | 0.0580 | 0.1001 | 0.1515 | 0.1081 |
| **B** | 1.2721 | 0.0803 | 0.0825 | 0.1505 | 0.0821 | 0.0919 | 0.1009 | 0.0723 |
| **C** | 0.1383 | 0.0812 | 0.1243 | 0.0937 | 0.0542 | 0.0641 | 0.0698 | 0.0702 |
| **D** | 0.1234 | 0.1198 | 0.1132 | 0.1396 | 0.3359 | 0.1201 | 0.2206 | 0.1281 |
| **E** | 0.0637 | 0.0960 | 0.1905 | 0.1152 | 0.0687 | 0.1284 | 0.1162 | 0.1692 |
| **F** | 0.0786 | 0.0916 | 0.0769 | 0.0998 | 0.0769 | 0.0707 | 0.1203 | 0.1461 |
| **G** | 0.1174 | 0.1667 | 0.1100 | 0.0998 | 0.0776 | 0.0958 | 0.1361 | 0.1184 |
| **H** | 0.0868 | 0.0540 | 0.1142 | 0.1774 | 0.1042 | 0.0610 | 0.1391 | 0.0880 |

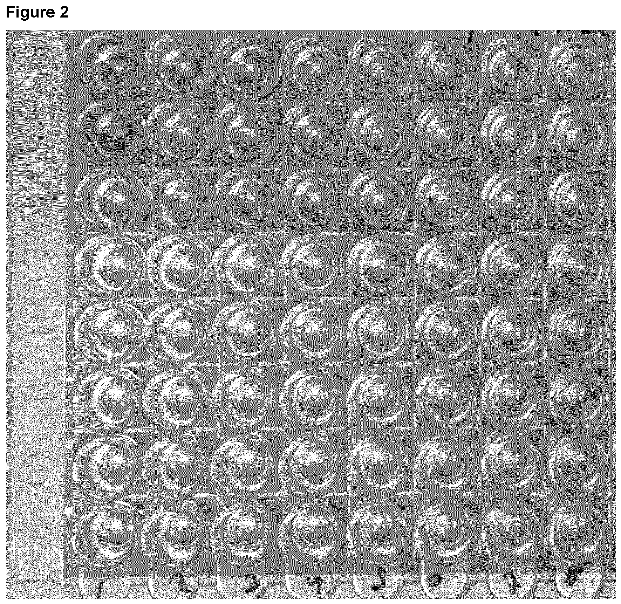

Corresponding to Figure 2 (ferrets)

### EXAMPLE 3

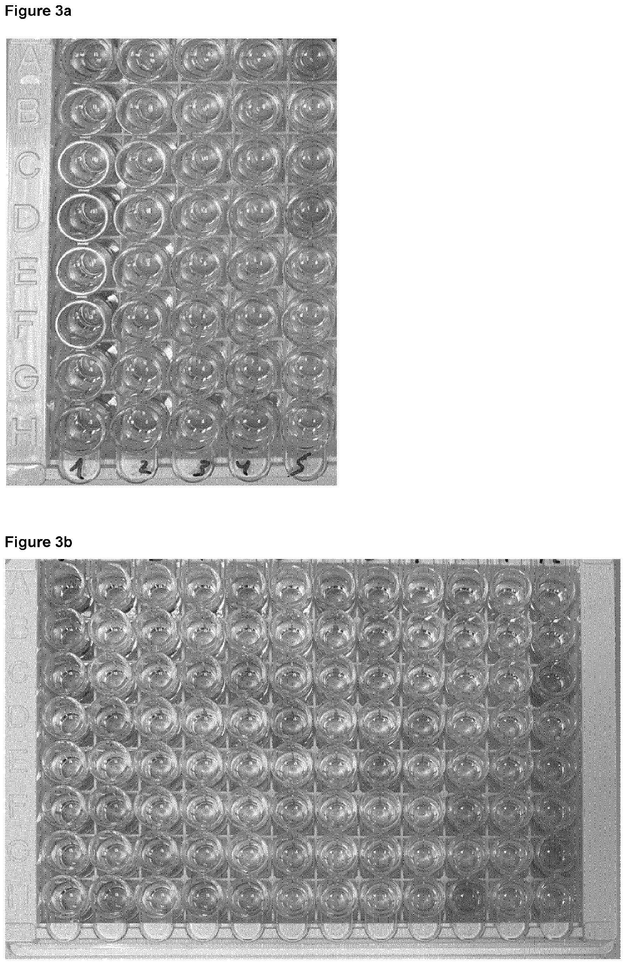

To evaluate the background signal of the ELISA of the present invention when human plasma is assayed, ELISAs were performed as detailed in Example 1 using human plasma from healthy individuals (n=24) and from SARS-CoV-2 PCR-positive patients (n=99). The results are shown in Figures 3a and Figure 3b, respectively. No background signal was observed in ELISA assays using seronegative human plasma, while the SARS-CoV-2 positive plasma samples showed high absorbance signal in all the samples tested, indicating that the ELISA of the present invention is able to detect anti-SARS-CoV-2 antibodies in human sera.

The ELISA's template was designed as follows:

| | 1 | 2 | 3 | 4 | 5 |
|---|---|---|---|---|---|
| A | SH+ | SH- | SH- | SH- | Human positive control 2 |
| B | SH+ | SH- | SH- | SH- | Human negative control 1 |
| C | SH+ | SH- | SH- | SH- | Human positive control 1 |
| D | SH+ | SH- | SH- | SH- | Human positive control 2 |
| E | SH+ | SH- | SH- | SH- | Human negative control 2 |
| F | SH+ | SH- | SH- | SH- | |
| G | SH- | SH- | SH- | SH- | |
| H | SH- | SH- | SH- | Human positive control 1 | |

Corresponding to Figure 3A

| | 1 | 2 | 3 | 4 | 5 | 6 | 7 | 8 | 9 | 10 | 11 | 12 |
|---|---|---|---|---|---|---|---|---|---|---|---|---|
| A | SH+ | SH+ | SH+ | SH+ | SH+ | SH+ | SH+ | SH+ | SH+ | SH+ | SH+ | SH+ |
| B | SH+ | SH+ | SH+ | SH+ | SH+ | SH+ | SH+ | SH+ | SH+ | SH+ | SH+ | SH+ |
| C | SH+ | SH+ | SH+ | SH+ | SH+ | SH+ | SH+ | SH+ | SH+ | SH+ | SH+ | SH+ |
| D | SH+ | SH+ | SH+ | SH+ | SH+ | SH+ | SH+ | SH+ | SH+ | SH+ | SH+ | SH+ |
| E | SH+ | SH+ | SH+ | SH+ | SH+ | SH+ | SH+ | SH+ | SH+ | SH+ | SH+ | SH+ |
| F | SH+ | SH+ | SH+ | SH+ | SH+ | SH+ | SH+ | SH+ | SH+ | SH+ | SH+ | Human positive control 1 |
| G | SH+ | SH+ | SH+ | SH+ | SH+ | SH+ | SH+ | SH+ | SH+ | SH+ | SH+ | Human positive control 2 |
| H | SH+ | SH+ | SH+ | SH+ | SH+ | SH+ | SH+ | SH+ | SH+ | SH+ | SH+ | Human negative control 1 |

Corresponding to Figure 3B

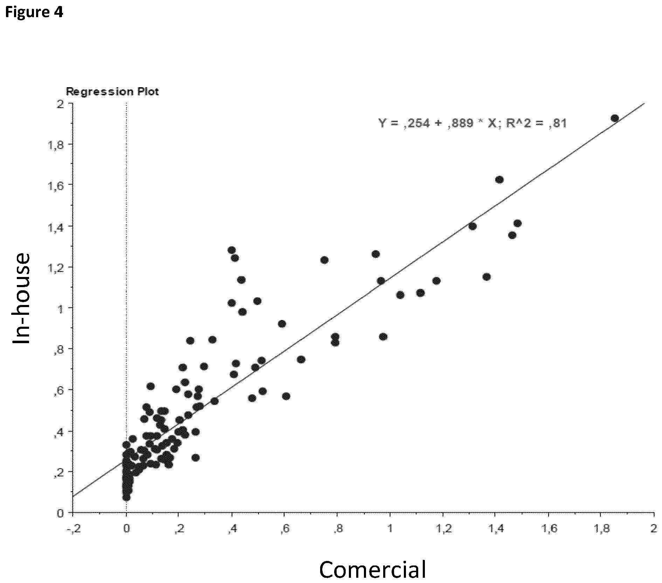

To further confirm the reliability of the ELISA of the present invention, a head-to-head comparison was performed between the ELISA of the present invention and a commercial ELISA assay (named SARS-CoV-2 S1RBD IgG ELISA Kit, obtained from MyBioSource) that only detects SARS-CoV-2 antibodies in human sera samples. The correlation between the absorbances obtained in the commercial ELISA test and the test of the present invention showed a coefficient of determination R² of 0.810 (Figure 4), indicating that the ELISA of the present invention is also accurate and correlates with commercially approved ELISA tests. The cut-off absorbance point calculated for the immunoassay developed herein to differentiate a positive sample from a negative sample in human plasma was 0.32 (mean+3 standard deviations of values from human samples pre-pandemic time) using StatView (SAS Institute, USA).

These results indicate that the assay of the present invention is, at least, as accurate as commercially approved ELISA tests that use human-specific secondary antibodies to detect antibodies in human sera.

### EXAMPLE 4

Next, to determine if Protein A performs better than other available conjugates, ELISAs were carried out following the same protocol as described in Example 1 but using Protein A or a commercially available Protein A/G conjugate. The absorbance developed in both tests was compared.

First, as a proof of concept, ferret sera from animals infected with *Leishmania* were used, and the results are shown in Figure 5a. ELISAS using Protein A developed higher absorbance in all cases (n= 8) than ELISAS using protein A/G. Interestingly, similar results were obtained when sera from humans, cats and ferrets infected with SARS-CoV-2 were assayed (Figure 5b; seronegative human sera and buffer without sera were used as control). It is noted that some of the cats and ferrets tested herein were in close contact with humans diagnosed as positive for SARS-CoV-2.

As shown in Figure 5b, the ELISA of the present invention using Protein A conjugate provided higher absorbance values than ELISAS using Protein A/G conjugate in positive samples, while absorbance values are similar in the case of negative samples. These findings are highly valuable because the ELISA of the present invention allows to distinguish negative from positive samples as the difference between the absorbances of negative and positive samples are higher when Protein A is used.

In conclusion, the ELISA of the present invention using Protein A conjugate provided greater color intensity than Protein A/G conjugate in serological assays for the determination of antibodies against *Leishmania* and SARS-CoV-2 in different animal samples, including human.

### EXAMPLE 5

Lastly, the absorbance correlation between ELISAs using Protein A and Protein A/G conjugates was analysed in 177 human sera, where 99 of them (n=99) were positive for SARS-CoV-2 and 78 were seronegative (n=78). High correlation was found between both ELISAs (Figure 6a, R² = 0.981), and statistical analyses were performed in order to find differences between the use of each conjugate (Protein A or Protein A/G) in the sensitivity of the ELISA test.

First, a t-Student test was performed with all sera samples (n=177), as indicated in Table 1 below:

**Table 1**

| **Unpaired t-test for TODOS** | | | | | | |
|---|---|---|---|---|---|---|
| **Grouping Variable: PROTEINA** | | | | | | |
| **Hypothesized Difference = 0** | | | | | | |
| | | Mean Diff. | | DF | t-Value | P-Value |
| PROT. A, PROT. A/G | | ,0441 | | 352 | ,669 | ,5040 |
| | | | | | | |

| **Group Info for TODOS** | | | | | | |
|---|---|---|---|---|---|---|
| **Grouping Variable: PROTEINA** | | | | | | |
| | Count | Mean | Variance | | Std. Dev. | Std. Err |
| PROT. A | 177 | ,527 | ,414 | | ,644 | ,048 |
| PROT. A/G | 177 | ,483 | ,339 | | ,582 | ,044 |

The results of this test indicate that no statistically differences (p=0.504) were found in the ELISAs using Protein A or Protein A/G conjugate when all samples are included in the analysis.

Next, positive and negative sera were analysed separately using two independent t-Student tests. The results are shown in Table 2 and 3. While there were no statistically differences between the use of Protein A or Protein A/G in sera samples positive for SARS-CoV-2 (Table 2, p=0.3391), statistical differences were found in seronegative samples (Table 3, p=0.0007).

**Table 2**

| **Unpaired t-test for Seropositivos** | | | | | |
|---|---|---|---|---|---|
| **Grouping Variable: Clase proteina** | | | | | |
| **Hypothesized Difference = 0** | | | | | |
| | | Mean | Diff. DF | t-Value | P-Value |
| Prot. A, Prot. A/G | | ,088 | 196 | ,958 | ,3391 |
| | | | | | |

| **Group Info for Seropositivos** | | | | | |
|---|---|---|---|---|---|
| **Grouping Variable: Clase proteina** | | | | | |
| | Count | Mean | Variance | Std. Dev. | Std. Err |
| Prot. A | 99 | ,886 | ,447 | ,669 | ,067 |
| Prot. A/G | 99 | ,799 | ,379 | ,615 | ,062 |

**Table 3**

| **Unpaired t-test for Seronegativos** | | | | | |
|---|---|---|---|---|---|
| **Grouping Variable: Clase proteina** | | | | | |
| **Hypothesized Difference = 0** | | | | | |
| | | Mean | Diff. DF | t-Value | P-Value |
| Prot. A, Prot. A/G | | -,012 | 154 | -3,441 | ,0007 |
| | | | | | |

| **Group Info for Seronegativos** | | | | | |
|---|---|---|---|---|---|
| **Grouping Variable: Clase proteina** | | | | | |
| | Count | Mean | Variance | Std. Dev. | Std. Err |
| Prot. A | 78 | ,070 | 3,701 E-4 | ,019 | ,002 |
| Prot. A/G | 78 | ,082 | ,001 | ,024 | ,003 |

These results indicate that the absorbance values in seronegative individuals in ELISAs using protein A/G are statistically higher than in ELISAs using Protein A. Therefore, ELISA tests using Protein A are more sensitive in seropositive animals since said ELISA presents the advantage of developing lower absorbance values in seronegative sera, allowing a better discrimination of the positive individuals with respect to the state of seronegativity and thus reducing the number of false negatives.

This is further corroborated when ROC values are obtained, as shown in Figure 6b, where the area under the curve is shown in Table 4 below:

**Area under the curve**

| Test Result Variable(s): | AREA | Standard error^{a} | Asymptotic significance^{b} | Asymptotic 95% Confidence interval | |
|---|---|---|---|---|---|
| | | | | Lower Bound | Upper Bound |
| Prot A | ,973 | ,013 | ,000 | ,947 | ,999 |
| Prot A/G | ,958 | ,017 | ,000 | ,924 | ,992 |

The test result variable(s): Prot A, Prot A/G has at least one the between the positive actual state group and the negative actual state group. Statistics may be biased.
a. Under the non-parametric assumption
b. Null hypothesis: true area = 0,5

Cut-off values obtained are:
**Protein A:** 0,1734 (sensitivity 92,9% and specificity 100%) considering Youden Index
**Protein A/G:** 0,1569 (sensitivity 91,9%, specificity 98,7%) considering Youden Index

This further demonstrates that ELISAs using Protein A according to the present invention have increased sensitivity and specificity than ELISAS using Protein A/G.

In conclusion, the present invention demonstrates that Protein A used in ELISAs allows not only a high throughput screening method that can be performed with sera from different animals, including human, but also that it provides a more sensible and specific and discriminative assay to differentiate positive SARS-CoV-2 sera samples, especially in the samples that present low absorbance values, for instance because they have low titter of antibodies.

## Claims

1. A multi-specie *in vitro* method for detecting in one biological sample an antibody that binds to at least one epitope of the SARS-CoV-2 virus, comprising:
a. contacting said at least one biological sample with at least one isolated SARS-CoV-2 protein, or at least one fragment of said isolated SARS-CoV-2 protein comprising at least one epitope of the SARS-CoV-2 virus, and
b. detecting the formation of an antigen-antibody complex between said virus protein or said fragment and an antibody present in said biological sample,
**characterized in that** the detection is performed by using a labelled protein, wherein the protein of the labelled protein consists of Protein A or a derivative thereof conjugated to a label.

2. The *in vitro* method of claim 1, wherein the at least one isolated SARS-CoV-2 protein is the SARS-CoV-2 Spike (S) protein of SEQ ID NO 1, or a variant or a fragment of SEQ ID NO 1 having at least 80% sequence identity to SEQ ID NO 1.

3. The *in vitro* method of claim 2, wherein the at least one fragment of said isolated SARS-CoV-2 S protein of SEQ ID NO 1 is the Receptor-binding domain (RBD) of SEQ ID NO 2, or a variant or a fragment of SEQ ID NO 2 having at least 80% sequence identity to SEQ ID NO 2.

4. The *in vitro* method of claim 3, wherein the RBD comprising at least one epitope of the SARS-CoV-2 virus, comprises at least 20 contiguous amino acid residues having at least 80% sequence identity with at least about 20 contiguous amino acid residues of SEQ ID NO. 2.

5. The *in vitro* method of any of claims 1 to 4, wherein said biological sample is a blood, plasma or serum sample.

6. The *in vitro* method of claim 5 wherein said biological sample is a blood, plasma or serum sample from a mammal, preferably a mammal selected from the group of human, mustelids or felines.

7. The *in vitro* method of any of the claims 5 and 6, wherein said biological sample is a serum sample, and said SARS-CoV-2 RBD is as set forth in SEQ ID NO 2.

8. The *in vitro* method of any of claims 1 to 7, wherein said method is an *in vitro* diagnostic method for the detection of a subject having antibodies against the SARS-CoV-2 virus, wherein said subject is diagnosed as having antibodies against the SARS-CoV-2 virus if an antigen-antibody complex between said virus protein, or said fragment, and an antibody present in said biological sample is detected.

9. The *in vitro* method of any of claims 1 to 8, wherein said method is a method for screening a mammal having antibodies against the SARS-CoV-2 virus from those not having antibodies against the SARS-CoV-2 virus, wherein the mammal is preferably a mammal selected from the group of human, mustelids or felines.

10. A multi-specie *in vitro* kit suitable for detecting in a biological sample an antibody that binds to at least one epitope of the SARS-CoV-2 virus comprising:
a. at least one isolated SARS-CoV-2 protein, or at least one fragment of said isolated SARS-CoV-2 protein comprising at least one epitope of the SARS-CoV-2 virus, and
b. reagents for detecting the formation of antigen-antibody complex between said at least one isolated SARS-CoV-2 protein, or a fragment thereof, and at least one antibody present in a biological sample,
**characterized in that** the detection is performed by using a labelled protein, wherein the protein of the labelled protein consists of Protein A or a derivative thereof conjugated to a label, and wherein said at least one isolated protein or fragment thereof and said reagents are present in an amount sufficient to detect the formation of said antigen-antibody complex.

11. The kit of claim 10, wherein said at least one isolated SARS-CoV-2 protein is the Spike (S) protein of SEQ ID NO 1 or a variant or a fragment of SEQ ID NO 1 having at least 80% sequence identity to SEQ ID NO 1.

12. The kit of any of the claims 10 and 11, wherein the at least one fragment of isolated SARS-CoV-2 S protein of SEQ ID NO 1 is the Receptor-binding domain (RBD) fragment of SEQ ID NO 2, or a variant or a fragment of SEQ ID NO 2 having at least 80% sequence identity to SEQ ID NO 2.

13. The kit of claim 12, wherein said at least one fragment of said isolated SARS-CoV-2 RBD comprising at least one epitope of the SARS-CoV-2 virus, is **characterized by** comprising at least 20 contiguous amino acid residues having at least 80% sequence identity with at least 20 contiguous amino acid residues of SEQ ID NO 2.

14. The kit of any of claims 10 to 13, wherein said at least one isolated SARS-CoV-2 RBD is the protein of SEQ ID NO 2.

15. The kit of any of claims 10 to 14, wherein said kit is an ELISA system comprising at least one isolated SARS-CoV-2 RBD of SEQ ID NO 2, to coat or coating a solid surface, preferably microtiter plate wells, and further consisting of Protein A conjugated to a label and one or more of the following reagents: blocking reagents for unbound sites to prevent false positive results; and substrates that react with the label, preferably the enzyme, to indicate a positive reaction.


SEQUENCE LISTING

<110> Universidad de Zaragoza
      Fundación Agencia Aragonesa para la Investigación y el
      Desarollo - ARAID
      Fundación Instituto de Investigación Sanitaria Aragón

<120> MULTI-SPECIES IMMUNOASSAYS FOR DETECTING
 ANTIBODIES ANTI-SARS-CoV-2

<130> 905 574

<160> 2

<170> BiSSAP 1.3.6

<210> 1
<211> 1273
<212> PRT
<213> Coronaviridae

<220>
<223> Amino acid sequence SARS-CoV-2 Spike protein

<400> 1

<210> 2

<211> 223
<212> PRT
<213> Coronaviridae

<220>
<223> Amino acid sequence SARS-CoV-2 RBD protein

<400> 2
Arg Val Gln Pro Thr Glu Ser Ile Val Arg Phe Pro Asn Ile Thr Asn
1               5                   10                  15
Leu Cys Pro Phe Gly Glu Val Phe Asn Ala Thr Arg Phe Ala Ser Val
            20                  25                  30
Tyr Ala Trp Asn Arg Lys Arg Ile Ser Asn Cys Val Ala Asp Tyr Ser
        35                  40                  45
Val Leu Tyr Asn Ser Ala Ser Phe Ser Thr Phe Lys Cys Tyr Gly Val
    50                  55                  60
Ser Pro Thr Lys Leu Asn Asp Leu Cys Phe Thr Asn Val Tyr Ala Asp
65                  70                  75                  80
Ser Phe Val Ile Arg Gly Asp Glu Val Arg Gln Ile Ala Pro Gly Gln
                85                  90                  95
Thr Gly Lys Ile Ala Asp Tyr Asn Tyr Lys Leu Pro Asp Asp Phe Thr
            100                 105                 110
Gly Cys Val Ile Ala Trp Asn Ser Asn Asn Leu Asp Ser Lys Val Gly
        115                 120                 125
Gly Asn Tyr Asn Tyr Leu Tyr Arg Leu Phe Arg Lys Ser Asn Leu Lys
    130                 135                 140
Pro Phe Glu Arg Asp Ile Ser Thr Glu Ile Tyr Gln Ala Gly Ser Thr
145                 150                 155                 160
Pro Cys Asn Gly Val Glu Gly Phe Asn Cys Tyr Phe Pro Leu Gln Ser
                165                 170                 175
Tyr Gly Phe Gln Pro Thr Asn Gly Val Gly Tyr Gln Pro Tyr Arg Val
            180                 185                 190
Val Val Leu Ser Phe Glu Leu Leu His Ala Pro Ala Thr Val Cys Gly
        195                 200                 205
Pro Lys Lys Ser Thr Asn Leu Val Lys Asn Lys Cys Val Asn Phe
    210                 215                 220